# EUROPEAN PATENT APPLICATION

(11) **EP 1 900 373 A1**
(43) Date of publication of application: **19.03.2008**
(21) Application number: 06019393.5
(22) Date of filing: 15.09.2006
(51) Int. Cl.: A61K 36/23, A61K 36/53, A61K 36/28, A61P 11/06

(54) **Composition for treating asthma**

(71) Applicant: Luay, Rashan, Shafa-Badran 11931 (JO); Fiebig, Heinz-Herbert, 79110 Freiburg (DE); Abdullah-Abdelhalim, Abo-Kadegha, Amman (JO)
(72) Inventor: Abo-Kadegha, Abdullah, Amman (JO); Sahban, Raya, Amman (JO); Rashan, Sara, Sult (JO); Rashan, Luay, Shafa Badran 11931 (JO); Soulimani, Rachid c/o Ethnobotanique et Pharma., 57040 Metz Cedex 01 F (FR); Fiebig, Heinz-Herbert, 79110 Freiburg (DE); Yahya Hamza Thanoon, Dubai (AE)
(74) Representative: Müller-Boré & Partner Patentanwälte

(57) **Abstract**

The present invention relates to a composition for treating asthma in an individual comprising extracts of aniseed, thymus and inula, a method for treating asthma in an individual comprising the step of administering said composition, and the use of said composition as a medicament.

## Description

The present invention relates to a composition for treating asthma in an individual comprising extracts of aniseed, thymus and inula, a method for treating asthma in an individual comprising the step of administering said composition, and the use of said composition as a medicament.

Asthma is a disease characterized by recurrent attacks of breathlessness and wheezing, which vary in severity and frequency from person to person. This condition is due to inflammation of the air passages in the lungs and affects the sensitivity of the nerve endings in the airways so they become easily irritated. In an attack, the lining of the passages swell causing the airways to narrow and reducing the flow of air in and out of the lungs. Asthma attacks all ages groups but often starts in childhood.

Asthma may either be a chronic respiratory impairment (hereinafter referred to as chronic asthma) or an intermittent illness marked by episodic symptoms that may result from a number of triggering events (hereinafter referred to as intermittent asthma). Such triggers may be exposure to environmental stimuli or allergens (allergic asthma), cold air, exercise or emotional stress. In particular, allergens may include waste from the house dust mite, grass pollen, mould spores and pet epithelial cells. Further, said triggers also may include medications such as aspirin or beta blockers, and air pollutants such as ozone, nitrogen dioxide and sulfur dioxide. In children, the most common triggers are viral illnesses such as those that cause the common cold. A further risk factor for asthma is a family history of asthma or allergy.

World-wide, between 100 and 150 million people suffer from asthma and this number is rapidly rising. For example, in Western Europe the number of individuals suffering from asthma has doubled in ten years. Deaths from this condition have reached over 180,000 annually around the globe. Further, asthma is not just a public health problem for developed countries. However, the incidence of the disease varies greatly in developing countries.

Asthma tends to occur in epidemics and very often affects young people. The human and economic burden associated with this conditions is severe. For example, the world-wide economic costs associated with asthma are estimated to exceed those of tuberculosis and HIV/AIDS combined.

The most effective treatment for asthma is identifying its triggers and limiting or avoiding exposure to them. Additionally, treatment is commonly based on suited medication. Specific treatments for asthma are broadly classified into relievers and preventers. Relief medication is generally achieved with fast acting bronchodilators which are provided in pocket-sized, metered-dose inhalers. Examples for such bronchodilators are selective beta₂-adrenoceptor agonists such as salbutamol, levalbuterol, terbutaline and bitolterol. Further, prevention medication are intended to suppress inflammation and to reduce the swelling of the lining of the airways. Examples for such prevention medication are inhaled glucocorticoids, leukotriene modifiers and mast cell stabilizers. Further, antihistamines are often used to treat allergic symptoms underlying the chronic inflammation.

However, most of the commonly used medicaments are based on active components that may cause undesired side effects in the treated individual.

Thus, the technical problem underlying the present invention is to provide a new system for treating asthma in an individual which is mild and highly acceptable for the treated individual.

The solution to the above technical problem is achieved by providing the embodiments characterized in the claims.

In particular, the present invention relates to a composition for treating asthma in an individual comprising extracts of aniseed, thymus and inula.

According to the present invention, the term "extract" means an aqueous extract. Said extract is obtainable by conventional methods known in the state of the art. Preferably, the extract is prepared by boiling a herbal mixture of aniseed, thymus and inula (hereinafter simply referred to as "herbal mixture") in water for 20 to 40 minutes, cooling at room temperature, preferably overnight, and filtering the cooled mixture. It is more preferred to prepare the extract by boiling 2 to 5 parts by weight of herbal mixture in 100 parts by weight of water for 25 to 30 minutes, cooling for 6 to 8 hours, and filtering the cooled mixture to obtain the aqueous extract. Alternatively, it is possible to prepare the aqueous extracts of each herb separately and to combine the respective aqueous extracts to form the aqueous extract of the herbal mixture.

According to the present invention, the term "aniseed" means *Pimpinella anisum* of the family Apiaceae which is also known as anise. Aniseed contains volatile oils which include anethole as main ingredient (up to 90 %). Further, the oils contain methyl chavicol, anisic aldehyde, mono- and sesquiterpenes.

According to the present invention, the term "thymus" means Garden Thyme *(Thymus vulgaris)* of the family Lamiaceae which is also known as Common Thyme. Thymus contains volatile oils which include thymol and carvacrol as main ingredients. The oils further contain p-cymene, 1,8-cineole, further mono- and triterpenes, flavonoides and antioxidatively active biphenyl compounds.

According to the present invention, the term "inula" means either elecampane *(Inula helenium)* or Inula viscosa of the family Asteraceae. Inula contains volatile oils which include helenin, polyacetylene, inuline and sequiterpene lactones such as alantolactone.

In a preferred embodiment of the present invention, the composition for treating asthma in an individual comprises 20 to 45 wt.% aniseed extracts, 20 to 45 wt.% thymus extracts and 20 to 45 wt.% inula extracts. Further, in a more preferred embodiment of the present invention, the composition for treating asthma in an individual comprises 35 wt.% aniseed extracts, 35 wt.% thymus extracts and 30 wt.% inula extracts. In another preferred embodiment, the composition for treating asthma in an individual comprises 5 wt.% aniseed extracts, 5 wt.% thymus extracts and 90 wt.% inula extracts.

Further, it should be noted that the term "individual" as used herein may be any mammal. In a preferred embodiment of the present invention, the individual is a human being.

The asthma to be treated with the composition according to the present invention may be any known form of asthma. Preferably, the asthma is obstructive bronchial asthma. More preferably, the asthma is chronic asthma or intermittent asthma resulting from a triggering event. The triggering event includes for example exposure to environmental stimuli or allergens (allergic asthma), cold air, exercise, viral illnesses or emotional stress. Examples for allergens include waste from the house dust mite, grass pollen, mould spores and pet epithelial cells. Further, said triggers also may include medications such as aspirin or beta blockers, and air pollutants such as ozone, nitrogen dioxide and sulfur dioxide.

The composition according to the present invention shows immune-modulatory properties through the release of IL-6, Interferon-gamma, TNF-alpha, and IL-10. In addition, the composition shows simultaneously potent bronchodilator characteristics and anti-inflammatory properties both *in vitro* and *in vivo.* Thus, the composition according to the present invention is suited for the relief treatment and the preventing treatment of asthma. Further, the composition according to the present invention has the advantage of being a safe and non-toxic herbal extract thereby having less side effects as compared to conventional compositions for the treatment of asthma.

The present invention further relates to a method of treating asthma in an individual comprising the step of administering the composition according to the present invention. In a preferred embodiment of the present invention, the composition is administered orally or respiratorily.

The composition according to the present invention is preferably administered in the form of an aqueous extract obtained from 1 to 5 g, more preferable from about 3 g of herbal mixture. In a preferred embodiment of the present invention the extract of the present invention is diluted 1 to 5 times before administration. In another preferred embodiment, the extract according to the present invention is further processed into capsules or tablets and is administered orally in one of said dosage forms. Suited methods for preparing capsules or tables from an aqueous extract are known in the state of the art. When being administered respiratorily, the aqueous extract is preferably applied by means of a pocket-sized, metered-dose inhaler in the form of an aerosol. It is further preferred to prepare a sterile lyophilized extract of the composition of the present invention.

According to the method of the present invention, the composition is preferably administered to the individual suffering from asthma regularly, i.e. independent of an asthma attack one to three times daily. Since the composition according to the present invention showed high anti-inflammatory properties, by said permanent administration a preventing treatment can be achieved. In another preferred embodiment, the composition of the present application is administered in the case of an acute asthma attack. Because of the potent bronchodilator characteristics of the composition of the present invention, said administration leads to a relieving effect of the asthma attack.

The composition for treating asthma according to the present invention has great advantages compared to conventional compositions for treating asthma. In particular, said composition is natural, safe, easy to deal with, with no serious side effects, cheap and leads to complete remission.

Additionally, the present invention relates to the use of the composition according to the present invention as a medicament. Preferably, the medicament contains one or more pharmaceutically acceptable carriers and/or diluents and/or auxiliary agents.

Moreover, the present invention relates to the use of the composition according to the present invention in the manufacture of a medicament for treating an individual suffering from asthma. In a preferred embodiment of the present invention said asthma is chronic asthma or intermittent asthma.

### Examples

### 1. Preparation of the extract

The extract was prepared by boiling 40 g of the herbal mixture comprising 5 wt.% aniseed extracts, 5 wt.% thymus extracts and 90 wt.% inula extracts in 2000 ml of water, cooling for 8 hours, filtering the cooled mixture and then lyophilizing the mixture under sterile conditions.

### 2. Study of toxicity of the extract of the present invention

### a) Acute toxicity:

The LD₅₀ of the above herbal extract was found to be more than 7 g/kg equal 21 g/m² using the oral and IP routes in albino balb/c mice and more than 10 g/kg equal 70 g/m² using the oral and the IP routes in albino rats. No apparent side effects were observed in both experimental animals except that both species showed depressant behaviours.

### b) Chronic toxicity:

The potential lethal effect of the extract of the invention was investigated chronically over a period of 8 months with an experimental design of three groups of 80 animals/group. Groups comprised male and female balbC/mice.

Group 1 received 1 g/kg daily orally with drinking water; group II received 3g/kg daily orally with drinking water, whereas, group III served as a control.

Morbidity and mortality checks were made daily. Body measurements were scheduled at day (0) and then on a monthly basis. A number of animals were sacrificed per month. Complete blood count (CBC), including leukocytes, differential cell count, erythrocytes, liver and kidney function tests were done on each sacrifice. Further, biopsies were taken from kidney, liver and intestine during each sacrifice for pathological investigations.

The following observations were recorded:
1) The extract of the present invention had no effects on the body weights increase in all groups.
2) The extract of the present invention showed no significant changes on CBC in all groups.
3) The extract of the present invention showed no significant changes in the ALT, AST, ALP enzyme activities, no changes in creatinine, and blood levels indicating no liver and kidney toxicity. Furthermore, no changes were observed in the total protein and total bilirubin in all the groups at the doses used, indicating that the extract of the invention had no effect on the liver and kidney functions.
4) Pathologically, no changes were observed in the organs studied both grossly and microscopically in the three groups.

Finally, the mutagenic effect of the extract of the present invention was also studied on two bacterial strains; *Salmonella typhimurium* TA1530 and *Salmonella typhimurum* TA1537 *in vitro* to see if the extract of the invention had any mutagenic effect. The results showed that the extract of the invention was safe and had no mutagenic effects on the two bacterial strains at concentrations up to 500 µg/ml.

### 3. Clinical studies

The above extract was administered to more than 2200 individuals suffering from Asthma. In more than 80% of the cases, a positive response to the treatment was observed.

Protocol for the treatment of asthma:
i. 300 to 400 ml of the herbal extract taken daily in 3 to 4 dividing doses; i.e. 100 ml is taken 3 to 4 times daily after meals and for a period of 4 to 6 months.
ii. One capsule (500 mg) containing the lyophilised material 3 times daily after meals and for a period of 4 to 6 months.

It is advisable to take the treatment continuously for a period of 4 to 6 months. For acute pulmonary asthma the treatment can be taken for one month only. It is recommended that a follow-up treatment for another two months is necessary for individuals suffering from chronic pulmonary asthma.

During the period between 1990 up to 2006, a total of more than 2,200 male and female patients (aged 2 to 65 years) suffering from chronic, intermittent and allergic asthma were treated with the extract of the invention. More than 90% of these patients had received high doses of anti-inflammatory as inhaled corticosteroids in combination with long acting B-agonist and extended-release theophilline or a leukotriene modifier.

In conclusion, the above clinical study which was conducted over a period of 16 years shows that the extract of the present invention is active to deal with patients suffering from acute to chronic obstructive pulmonary asthma and allergic asthma. More than 80% of the treated individuals showed a complete response to the extract of the invention. The early signs of responding to this treatment begins after 72 to 96 hours following treatment with the extract of the invention. However, satisfactory results appear after 2 to 3 weeks following treatment with the extract of the invention especially in adult individuals suffering from chronic obstructive pulmonary asthma. However, in immature individuals, the response to the extract of the invention was rather different from mature individuals. It has been observed that children below 16 years responded quickly to the treatment and, therefore, they showed very good satisfactory results following 1 to 2 weeks after the treatment and they recovered in a period of about 3 months from chronic obstructive pulmonary asthma, whereas, mature individuals required more time to recover and the duration of the treatment could reach up to 6 months or even more. Further, young individuals aged 2 to 8 years on the other hand, showed the highest rate of responding to the extract of the invention compared to others.

No toxic or serious side effects were observed during these studies. However, a number of side effects were recorded in some patients including nausea, which appeared in about 2 to 3% of the individuals treated with the extract of the invention. This indeed can be mainly attributed to the bad taste (bitterness) of the extract of the invention. This nausea was accompanied by vomiting in 1% of the individuals who used the extract of the invention. Diarrhoea and abdominal gases were also recorded in 3 to 4% of the patients who used the extract of the invention. Urination was recorded in more than 7% of the individuals and this was mostly due to some components present in the inula. More than 30% of the individuals who used the treatment of the present invention showed expectorant effect and felt that breathing was improved especially in smoking individuals.

It is noted at this stage that regular follow-up had been performed on a large number of these individuals treated with the extract of the invention. Different scans such as CT, X-ray, lung function and physical examination were used in the follow-up. The follow-up had been continued in some individuals for more than 8 years and under the supervision of medical doctors.

In conclusion, the clinical studies performed on patients suffering from different types of pulmonary asthma showed that the extract of the present invention was active, safe, non-toxic and possessed some immune-stimulatory characteristics with potent bronchodilator and anti-inflammatory properties and this was clearly indicated by the ability of the mixture to effectively reduce the two main components of the asthma disease, i.e. bronchial hyperresponsiveness and airways inflammation.

## Claims

1. A composition for treating asthma in an individual comprising extracts of aniseed, thymus and inula.

2. The composition according to claim 1, comprising 20 to 45 wt.% aniseed extracts, 20 to 45 wt.% thymus extracts and 20 to 45 wt.% inula extracts.

3. The composition according to claim 1 or 2, comprising 35 wt.% aniseed extracts, 35 wt.% thymus extracts and 30 wt.% inula extracts.

4. The composition according to claim 1, comprising 5 wt.% aniseed extracts, 5 wt.% thymus extracts and 90 wt.% inula extracts.

5. The composition according to anyone of claims 1 to 4, wherein the individual is a human being.

6. The composition according to anyone of claims 1 to 5, wherein the asthma is chronic asthma or intermittent asthma.

7. A method of treating asthma in an individual comprising the step of administering the composition according to anyone of claims 1 to 6 to said individual.

8. The method according to claim 7, wherein the composition is administered orally or respiratorily.

9. Use of a composition according to any one of claims 1 to 6 as a medicament.

10. Use of a composition according to any one of claims 1 to 6 in the manufacture of a medicament for treating an individual suffering from asthma.

11. Use according to claim 10, wherein the asthma is chronic asthma or intermittent asthma.
